# EUROPEAN PATENT APPLICATION

(11) **EP 4 681 771 A1**
(43) Date of publication of application: **21.01.2026**
(21) Application number: 24902192.4
(22) Date of filing: 22.08.2024
(51) Int. Cl.: A61N 5/06

(54) **BEAUTY DEVICE**

(30) Priority: 12.12.2023 CN 202323389575 U
(71) Applicant: Shenzhen Zhuolingkang Technology Co., Ltd, Shenzhen, Guangdong 518000 (CN)
(72) Inventor: ZENG, Weijun, Shenzhen, Guangdong 518000 (CN)
(74) Representative: Studio Torta S.p.A.
(86) International application number: PCT/CN2024/113894
(87) International publication number: WO 2025/123766

(57) **Abstract**

Disclosed is a beauty device (10), comprising a body (100) and a wearable accessory (200). The body (100) is provided with a power supply component and a light-emitting component (110), the power supply component being configured to supply power, and the light-emitting component (110) comprising a pulsed light source capable of emitting pulsed light. The wearable accessory (200) comprises a housing (210) adapted to be worn on a corresponding part of the human body, with a cavity being formed inside the housing (210) for allowing the corresponding part of the human body to access. The housing (210) is provided with at least one light inlet (211), the body (100) is detachably connected to the light inlet (211) of the housing (210), and the pulsed light emitted by the pulsed light source enters the cavity inside the housing (210) through the light inlet (211) to act on the human skin. By means of the provision of the wearable accessory (200) that is detachably connected to the body (100), the beauty device (10) can be adapted to be worn on the corresponding part of the human body for beauty treatment, thereby allowing a user to free up the hands thereof, and making the beauty device (10) more convenient and effortless to use.

## Description

### CROSS-REFERENCE TO RELATED APPLICATIONS

The present application claims priority to Chinese Patent Application No. 2023233895750, titled "Beauty Device", filed on December 12, 2023, the entire disclosure of which is hereby incorporated herein by reference.

### TECHNICAL FIELD

The present application relates to the technical field of beauty equipment, and in particular to a beauty device.

### BACKGROUND

A beauty device, as a device with functions such as freckle removal, reduction or elimination of wrinkles, improvement of skin condition, and removal of body hair on the skin surface, is increasingly favored by people, and the requirements for it are also getting higher and higher. Existing beauty devices are mainly composed of a lamp head and a body. The lamp head generally includes a pulsed light source, which directly irradiates the skin with intense pulsed light emitted by the pulsed light source, causing photobiochemical effects on the skin to achieve corresponding cosmetic effects. The body provides power and control signals for the lamp head.

Affected by the area of the light outlet (generally 1-4 square centimeters), the irradiation area of photons of existing handheld beauty devices moves with the movement of the instrument during use. Therefore, users need to hold and operate the beauty device to make it irradiate the skin area requiring beauty treatment. However, long-term handheld operation will cause hand fatigue, thereby deteriorating the user experience.

### SUMMARY

One object of the present application is to solve the problem in the related art that long-term handheld operation causes hand fatigue of users, thereby deteriorating the user experience, and to provide a beauty device that is convenient to use. To solve the above technical problems, the present application adopts the following technical solutions:

A beauty device includes:
a main body provided with a power supply component for power supply and a light-emitting component comprising a pulsed light source for emitting pulsed light; and
a wearable accessory comprising a housing adapted to be worn on a corresponding part of a human body;
wherein a cavity for the corresponding part of the human body to enter is formed inside the housing, and at least one light inlet is provided on the housing; and
the main body is detachably connected to the at least one light inlet of the housing, and pulsed light emitted by the pulsed light source enters the cavity inside the housing through the at least one light inlet to act on a human skin.

In one embodiment, the beauty device further includes an electrical connection assembly provided on a surface where the main body and the housing are docked;
the electrical connection assembly includes a first electrical connection portion and a second electrical connection portion that are electrically connected in a matching manner; and
the first electrical connection portion is provided on one of the main body and the housing, and the second electrical connection portion is provided on another of the main body and the housing.

In one embodiment, the beauty device further includes:
a heating module provided inside the housing for raising a temperature of the human skin;
a temperature sensor element provided on a surface of the housing corresponding to the human skin for sensing skin temperature; and
an image acquisition module provided inside the housing for acquiring skin state information;
wherein the heating module, the temperature sensor element, and the image acquisition module are electrically connected to the power supply component on the main body through the electrical connection assembly.

In one embodiment, the beauty device further includes a snap assembly for snapping and fixing the main body and the housing;
wherein the snap assembly includes a snap-fit portion and a snap-in portion that are in snapping fit;
the snap-fit portion is provided on one of the main body and the housing, and the snap-in portion is provided on another of the main body and the housing.

In one embodiment, the snap-fit portion is a snap seat protruding from a surface of the housing, the snap seat is provided with a plug-in slot perpendicular to each light inlet, and a snap protrusion is protruded from an inner wall of the plug-in slot; and
the snap-fit portion is a snap groove opened on a circumferential side wall of the main body, and when the main body is inserted into the snap seat along the plug-in slot, the snap protrusion is snapped in the snap groove.

In one embodiment, the snap-fit portion is a snap seat protruding from a surface of the housing, one side of the snap seat is open to form a sliding groove parallel to each light inlet, and a snap protrusion is protruded from an inner wall of the sliding groove; and
the snap-in portion is a snap groove opened on a circumferential side wall of the main body, and when the main body slides into the snap seat along the sliding groove, and the snap protrusion is snapped in the snap groove.

In one embodiment, a limitation portion is provided on the housing, and located at an opening of the snap seat, for limiting the main body in the sliding groove.

In one embodiment, a reflective layer is provided on a surface of the housing corresponding to the human skin, and distributed around each light inlet.

In one embodiment, a filter is detachably provided at each light inlet of the housing, and the pulsed light emitted by the pulsed light source is transmitted through the filter.

In one embodiment, the beauty device further includes an LED light source provided on a surface of the housing corresponding to the human skin.

In one embodiment, the beauty device further includes an LED light source provided on the main body and capable of extending into the housing through each light inlet.

In one embodiment, the housing includes a housing body and an abutting portion provided on a circumferential edge of the housing body, the abutting portion is integrally connected with the housing body, and the abutting portion is configured to make the cavity inside the housing relatively closed when the wearable accessory is worn on the corresponding part of the human body.

In one embodiment, a binding member is provided on the housing, and detachably connected to the housing, for fixing the wearable accessory to the corresponding part of the human body.

In one embodiment, a binding member is provided on the housing, and fixed to the housing, for fixing the wearable accessory to the corresponding part of the human body.

In one embodiment, the wearable accessory is configured to cover at least one form of skin on a face, a neck, a limbs, an abdomen, and a back.

**In** one embodiment, each of a plurality of light inlets is provided corresponding to different skin areas of the human body.

**In** one embodiment, the main body is of an integrated structure.

**In** one embodiment, the main body is of a split structure, and the power supply component and the light-emitting component are provided separately.

**It** can be seen from the above technical solutions that the present application has at least the following advantages and positive effects:

**In** the present application, by providing a wearable accessory detachably connected to the main body, the beauty device can be adapted to be worn on a corresponding part of the human body for beauty treatment, thereby freeing the user's hands and making the beauty device more convenient and labor-saving to use. Moreover, the wearable accessory can be configured into various structures capable of covering any part of the body. Therefore, the user can select a wearable accessory with a corresponding structure, connect it to the main body, and then perform beauty treatment on the corresponding part of the body, which is convenient to use and has a good beauty effect.

### BRIEF DESCRIPTION OF THE DRAWINGS

FIG. 1 is a structural schematic view of a beauty device according to an embodiment.
FIG. 2 is a first structural schematic view of a wearable accessory as a face mask according to the embodiment.
FIG. 3 is a second structural schematic view of a wearable accessory as a face mask according to the embodiment.
FIG. 4 is a structural schematic view of a wearable accessory as a hand beauty cover according to the embodiment.
FIG. 5 is an exploded structural schematic view of the beauty device shown in FIG. 1.
FIG. 6 is a structural schematic view of a beauty device according to another embodiment.
FIG. 7 is a structural schematic view of a beauty device according to yet another embodiment.
FIG. 8 is a schematic view of a main body with a split structure according to the embodiment.

The reference numerals:
10: Beauty device;
100: Main body; 110: Light-emitting component;
120: First housing; 130: Second housing; 140: External thread; 150: Connection cable; 200: Wearable accessory;
210: Housing; 211: Light inlet; 212: Housing body; 213: Abutting portion; 214: Internal thread;
220: Reflective layer; 230: Heating module; 231: Heating element; 232: Fan;
240: Temperature sensor element; 250: Binding member; 260: Lamp panel;
300: Electrical connection assembly; 310: First electrical connection portion; 320: Second electrical connection portion; 400: Snap assembly; 410: Snap seat; 411: Plug-in Slot; 412: Snap protrusion; 413: Sliding groove; 420: Snap groove; 430: Limitation portion.

### DETAILED DESCRIPTION OF THE EMBODIMENTS

The present application provides a beauty device. To make the objectives, technical solutions, and effects of the present application clearer and more explicit, the present application is further described in detail below with reference to the accompanying drawings and embodiments. It should be understood that the specific embodiments described herein are only used to explain the present application, and are not used to limit the present application.

For traditional handheld beauty devices, when performing beauty treatment on corresponding parts of the human body, users need to continuously move the beauty device to achieve complete irradiation of the skin of the part. Therefore, users usually need to spend a lot of time to complete a beauty treatment for the entire face, limbs, or other parts, which is time-consuming and laborious. In addition, when the beauty device is used, the light-emitting irradiation area moves with the movement of the user. The user cannot ensure uniform movement, so that some skin is easily irradiated repeatedly, while some skin is not irradiated, resulting in uneven skin beauty and poor beauty effect. The present application aims to provide a beauty device that is convenient to use and has a good beauty effect.

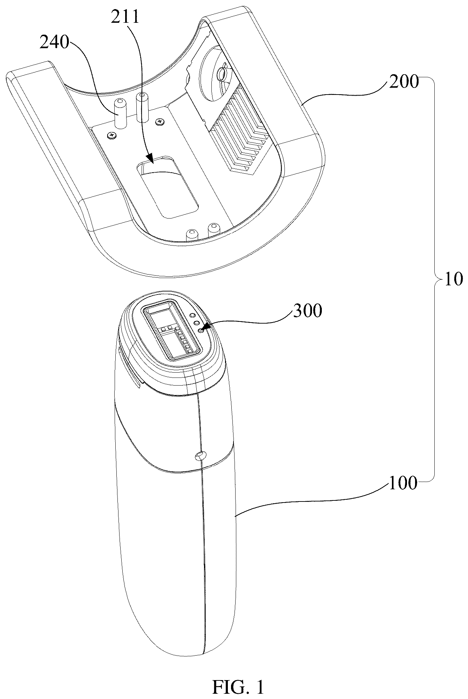

Please refer to FIG. 1. The beauty device 10 according to the embodiment of the present application includes a main body 100 and a wearable accessory 200. The main body 100 is provided with a power supply component and a light-emitting component 110. The power supply component is used for power supply, and the light-emitting component 110 includes a pulsed light source capable of emitting pulsed light. The wearable accessory 200 includes a housing 210 adapted to be worn on a corresponding part of the human body, and a cavity for the corresponding part of the human body to enter is formed inside the housing 210. The housing 210 is provided with at least one light inlet 211. The main body 100 is detachably connected to the light inlet 211 of the housing 210, and the pulsed light emitted by the pulsed light source enters the cavity inside the housing 210 through the light inlet 211 and acts on the human skin. By setting the wearable accessory 200 detachably connected to the main body 100, the beauty device 10 can be adapted to be worn on the corresponding part of the human body for beauty treatment. Compared with the traditional handheld beauty device, it does not need to move from one area of the corresponding part of the human body to another area during the beauty process, but can complete the beauty treatment of the part at one time, thereby not only saving greatly time, but also freeing the user's hands, making it more convenient and labor-saving to use. In addition, the wearable accessory 200 can be configured into various structures that can cover any part of the body. Therefore, the user can select the wearable accessory 200 with the corresponding structure, connect it with the main body 100, and then perform beauty treatment on the corresponding part of the human body, which is flexible to use and has strong applicability.

The housing 210 of the wearable accessory 200 is provided with at least one light inlet 211. It can be understood that when the wearable accessory 200 is configured to perform beauty treatment on a human part with a large area, such as the face, abdomen, or back, a plurality of the light inlets 211 can be provided on the housing 210 to achieve the purpose of increasing the light-emitting area, so that the light emitted by the wearable accessory 200 can cover all the skin of the beauty part. Or, when the wearable accessory 200 is configured to perform beauty treatment on a human part with a small area, such as the hand, one or two light inlet 211 on the housing 210 can be provided, as long as the light emitted by the wearable accessory 200 can cover all the skin of the beauty part.

In one embodiment, the wearable accessory 200 is configured to cover at least one form of skin on the face, neck, limbs, abdomen, and back. For the beauty device 10 of the present application, the user can select the wearable accessory 200 of the corresponding form as needed and connect it with the main body 100, so that the user can perform light irradiation beauty treatment on the corresponding part that needs beauty treatment.

In addition, the beauty device 10 of the present application can cover and irradiate all areas of the face, all areas of the neck, all areas of the back, etc. at one time, thereby maximizing the consistency of skin beauty treatment on the corresponding part of the human body and greatly improving the overall beauty effect of the skin.

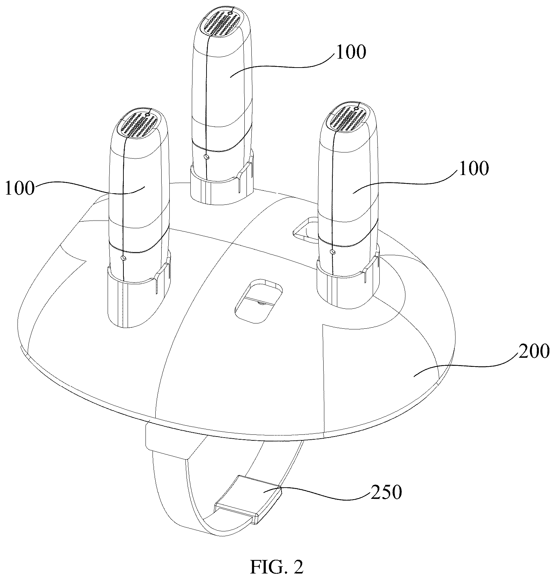

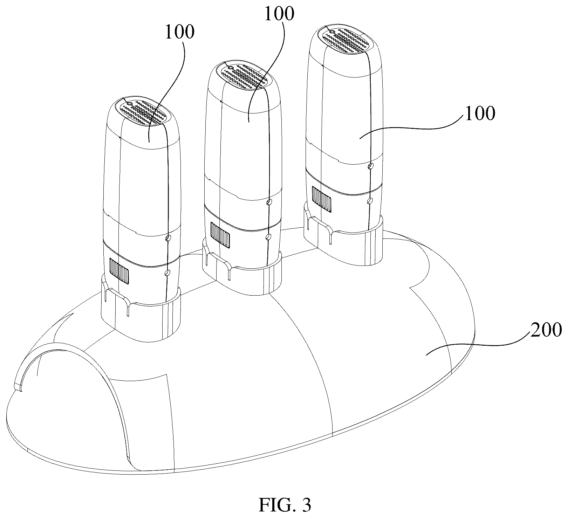

Optionally, a plurality of the light inlets 211 are provided, and each light inlet 211 is respectively set corresponding to different skin areas of the human body. Specifically, refer to FIGS. 2 and 3. Both FIGS. 2 and 3 illustrate structural schematic views of the wearable accessory 200 as a face mask for human face care.

As shown in FIGS. 2 and 3, the wearable accessory 200 is configured as a face mask that can cover the entire face. There are a plurality of the light inlets 211, and each light inlet 211 is provided on the face mask corresponding to the two cheek areas, the forehead area, the mid-face area, and the chin area of the human face.

Specifically, as shown in FIG. 2, the face mask is provided with three light inlets 211, which are respectively set corresponding to the two cheek areas and the forehead area. A main body 100 is respectively connected to each light inlet 211, so that the emitted light can cover the entire face.

Or, as shown in FIG. 3, the face mask is provided with three light inlets 211, which are respectively set corresponding to the forehead area, the mid-face area, and the chin area. All light inlet 211 is connected to one main body 100 , so that the emitted light can cover the entire face.

In other embodiments, the face mask may also be provided with only one or two, or four or five light inlets 211. One light inlet 211 may be provided in the mid-face area corresponding to the human face on the face mask. Two light inlets 211 may be provided in the two cheek areas corresponding to the human face on the face mask. Four light inlets 211 may be provided in the two cheek areas, the forehead area, and the chin area corresponding to the human face on the face mask. Five light inlets 211 may be provided in the two cheek areas, the forehead area, the mid-face area, and the chin area corresponding to the human face on the face mask.

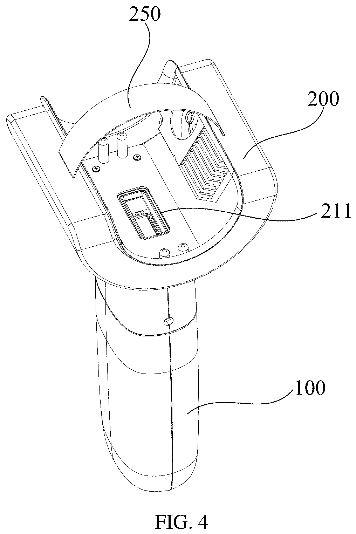

FIG. 4 is a schematic diagram of the wearable accessory 200 as a hand beauty cover for human hand care. As shown in FIG. 4, the wearable accessory 200 is configured as a hand beauty cover that can cover the hand. The hand beauty cover has an arc-shaped housing, and a light inlet 211 is opened in the middle of the arc-shaped housing. A main body 100 is connected to the light inlet 211, thereby performing beauty treatment on the hand.

In other embodiments, the wearable accessory 200 may also be designed in the form of a vest, etc., and a plurality of light inlets 211 are respectively provided corresponding to the chest, abdomen, and back areas of the human body to respectively connect a plurality of main bodies 100, thereby performing beauty treatment on the chest, abdomen, and back of the human body.

Referring to FIGS. 2 and 4, in one embodiment, the beauty device 10 further includes a binding member 250. The binding member 250 is detachably or fixedly connected to the housing 210, and the binding member 250 can fix the wearable accessory 200 to the corresponding part of the human body. The binding member 250 may be in various structural forms such as an elastic band or a telescopic band. Or, the binding member 250 may be two sections of binding bands connected by a snap-fit structure, and each section of the binding band is respectively and fixedly connected to the housing 210.

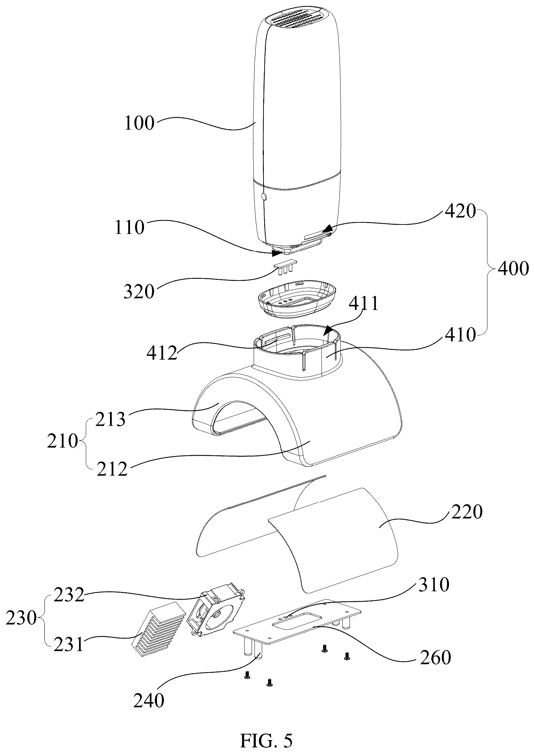

Referring to FIG. 5, in one embodiment, the housing 210 includes a housing body 212 and an abutting portion 213 provided on the circumferential edge of the housing body 212. The abutting portion 213 is integrally connected with the housing body 212, and the abutting portion 213 is used to make the cavity inside the housing 210 relatively closed when the wearable accessory 200 is worn on the corresponding part of the human body. In this embodiment, the housing body 212 may be made of a hard material, which is convenient for forming a shape approximately conforming to the face, neck, and other parts of the human body. The abutting portion 213 may be made of a flexible material, which is convenient for closely contacting the skin surface of the human body to play a role in sealing and preventing light leakage.

As shown in FIG. 5, in one embodiment, a reflective layer 220 is provided on the surface of the housing 210 corresponding to the human skin, and the reflective layer 220 is distributed around each light inlet 211. By setting the reflective layer 220, the light emitted from the light inlet 211 can be reflected, so that the light can act on the skin of the corresponding part of the human body more efficiently and widely.

In one embodiment, the beauty device 10 further includes a filter, which is detachably provided at the light inlets 211 of the housing 210, and the pulsed light emitted by the pulsed light source is transmitted through the filter. By setting the filter, it is possible to allow light of a specific wavelength to pass through and filter out light of other wavelengths, so that the light finally acting on the human skin is light of a specific band suitable for skin treatment or beauty, thereby expanding the efficacy of the beauty device 10.

In the embodiment of the present application, the filter is preferably detachably mounted at the light inlet 211 of the housing 210, and different beauty needs are met by replacing the filter. For example, a plug-in slot may be provided at the light inlet 211 of the housing 210, and the filter can be inserted into the plug-in slot. Or, the filter may be detachably attracted to the light inlet 211 of the housing 210 by using a magnetic attraction structure.

Referring to FIG. 5, in one embodiment, the beauty device 10 further includes an LED light source, which is provided on the surface of the housing 210 corresponding to the human skin. The LED light source includes a lamp panel 260 and a plurality of LED lamp beads provided on the lamp panel 260. The LED lamp beads may include red LED lamp beads, blue LED lamp beads, and green LED lamp beads. In the present application, the distribution and setting form of each LED lamp bead are not specifically limited, and each LED lamp bead may be set in a divided area or mixed arrangement according to needs in a certain rule, as long as the purpose of achieving a better beauty effect is achieved.

Certainly, in other embodiments, the LED light source may also be provided on the main body 100 and can extend into the housing 210 through the light inlet 211. Specifically, the LED light source may be provided on the lamp head part of the main body 100.

Referring to FIG. 5, in one embodiment, the beauty device 10 further includes a heating module 230, which is provided inside the housing 210 and is used to raise the temperature of the human skin. By setting the heating module 230, the air in the cavity inside the housing 210 can be heated, so that the temperature of the human skin can be raised, the pores of the human skin can be opened, and the beauty effect can be improved.

Optionally, the heating module 230 includes a heating element 231, which is used for heating. The heating element 231 may be a heating wire, a heating sheet, or an infrared heater, etc.

Optionally, the heating module 230 may further include a fan 232, which is used to realize the circulation of the air in the cavity inside the housing 210, so as to uniformly heat the skin of the corresponding part of the human body.

Referring to FIG. 5, in one embodiment, the beauty device 10 further includes a temperature sensor element 240, which is provided on the surface of the housing 210 corresponding to the human skin and is used to sense the skin temperature. Specifically, the temperature sensor element 240 protrudes from the inner surface of the housing 210. The temperature sensor element 240 may be a contact temperature sensor such as a thermistor or a thermocouple, which detects the temperature of the user's skin by contacting the human skin.

In other embodiments, the temperature sensor element 240 may also be a non-contact temperature sensor, such as an optical pyrometer, etc.

By setting the temperature sensor element 240, the skin temperature of the user can be detected in real time, so that when the detected temperature exceeds the preset value, the beauty device 10 is controlled to stop working to prevent the user's skin from being scalded due to excessive temperature. Or, the beauty device 10 can be controlled to enter different working modes according to the detected temperature information. For example, when the temperature value does not reach the appropriate temperature, the beauty device 10 is kept in the heating mode. When the temperature value reaches the appropriate temperature, the beauty device 10 enters the beauty mode, etc.

In other embodiments, the beauty device 10 further includes an image acquisition module, which is provided inside the housing 210 and is used to acquire the skin state information. The image acquisition module may be a camera or the like, which can acquire the skin state information, so as to facilitate targeted beauty care.

In the present application, the heating module 230, the temperature sensor element 240, and the image acquisition module are all electrically connected to the power supply component on the main body 100 through the electrical connection assembly 300 described below. The connection and cooperation relationship of the wearable accessory 200 and the main body 100 will be specifically described below in combination with the following embodiments.

Correspondingly, the main body 100 of the beauty device 10 is mainly used to realize the functions of power supply, pulsed light emission, and corresponding control of the whole machine. The main body 100 may adopt the beauty device 10 in the related art, as long as it can realize the aforementioned main functions. The main body 100 is not limited to a lamp head and body structure that are integrally formed, and may also be a lamp head and body structure that are separate, which is specifically described later.

In the present application, the housing 210 of the wearable accessory 200 may be mechanically connected to the main body 100 by using various structures that can realize detachable fixed connection, such as a threaded locking structure, a snapping and buckling structure, an interference fit structure, or a magnetic attraction structure, etc.

Referring to FIG. 5, in one embodiment, the beauty device 10 further includes a snap assembly 400, and the main body 100 and the housing 210 are snapped and fixed by the snap assembly 400. The snap assembly 400 includes a snap-fit portion and a snap-in portion that are in snap fit. The snap-fit portion is provided on one of the main body 100 and the housing 210, and the snap-in portion is provided on another of the main body 100 and the housing 210. The snap assembly 400 can facilitate and quickly realize the connection of the main body 100 and the housing 210, which is more convenient for users.

Optionally, as shown in FIG. 5, the snap-fit portion is a snap seat 410 protruding from the surface of the housing 210. The snap seat 410 is formed with a plug-in slot 411 perpendicular to the light inlet 211, and a snap protrusion 412 protrudes from the inner wall of the plug-in slot 411. The snap-in portion is a snap groove 420 opened on a circumferential side wall of the main body 100. When the main body 100 is inserted into the snap seat 410 along the plug-in slot 411, the snap protrusion 412 is snapped in the snap groove 420.

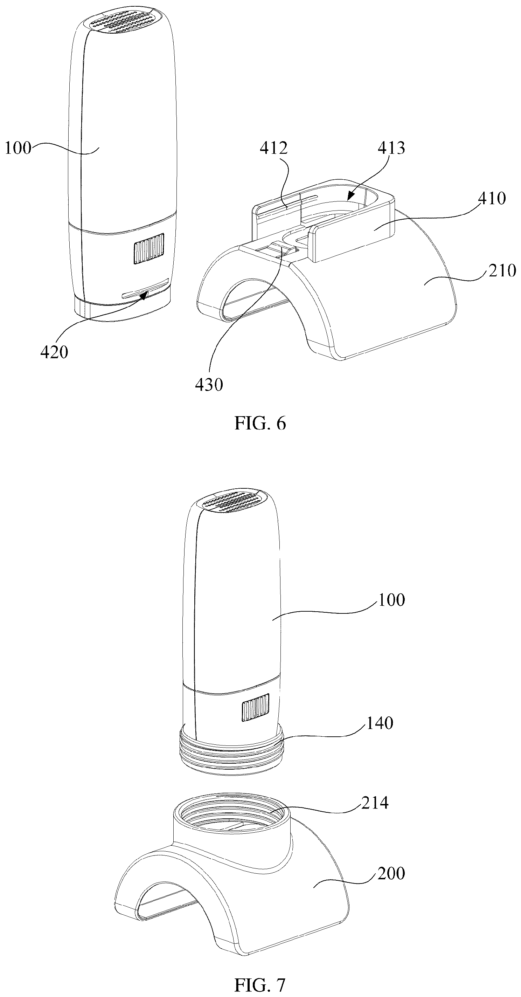

Referring to FIG. 6, in another embodiment, the snap-fit portion is a snap seat 410 protruding from the surface of the housing 210. One side of the snap seat 410 is open to form a sliding groove 413 parallel to the light inlet 211, and a snap protrusion 412 protrudes from the inner wall of the sliding groove 413. The snap-in portion is a snap groove 420 opened on the circumferential side wall of the main body 100. When the main body 100 slides into the snap seat 410 along the sliding groove 413, the snap protrusion 412 is snapped in the snap groove 420.

Optionally, the housing 210 is further provided with a limitation portion 430, which is located at the opening of the snap seat 410 and can limit the main body 100 in the sliding groove 413. As shown in FIG. 6, the limitation portion 430 is an elastic protrusion provided at the opening of the snap seat 410. In the process that the main body 100 slides into the snap seat 410 along the sliding groove 413, the elastic protrusion elastically deforms downward to facilitate the sliding of the main body 100. When the main body 100 is completely slid into the sliding groove 413, the elastic protrusion is reset to abut against the main body 100, thereby effectively preventing the main body 100 from slipping out of the sliding groove 413.

In other embodiments, as shown in FIG. 7, the parts where the housing 210 and the main body 100 are docked is respectively provided with an internal thread 214 and an external thread 140 that can be in threaded fit, and the housing 210 and the main body 100 are detachably connected through the internal thread 214 and the external thread 140.

In one embodiment, the beauty device 10 further includes an electrical connection assembly 300, which is provided on the surfaces where the main body 100 and the housing 210 are docked. As shown in FIG. 5, the electrical connection assembly 300 is a first electrical connection portion 310 and a second electrical connection portion 320 that are electrically connected in a matching manner. The first electrical connection portion 310 is provided on one of the main body 100 and the housing 210, and the second electrical connection portion 320 is provided on another of the main body 100 and the housing 210. By setting the electrical connection assembly 300, the electrical connection of the wearable accessory 200 and the main body 100 can be realized.

Optionally, the first electrical connection portion 310 and the second electrical connection portion 320 may be contact-type electrical contacts that can be electrically connected in a matching manner. When the main body 100 and the housing 210 are connected together, the wearable accessory 200 and the main body 100 are electrically connected through the electrical contacts on the main body 100 and the electrical contacts on the housing 210.

In other embodiments, the first electrical connection portion 310 and the second electrical connection portion 320 may also be plug-in terminals that can be electrically connected in a matching manner. When the main body 100 and the housing 210 are connected together, the wearable accessory 200 and the main body 100 are electrically connected through the terminals on the main body 100 and the terminal blocks on the housing 210.

The main body 100 of the beauty device 10 in the embodiment of the present application may be an integrated structure. As shown in FIG. 1, the main body 100 includes a substantially cylindrical housing, and the power supply component and the light-emitting component 110 are both provided in the housing.

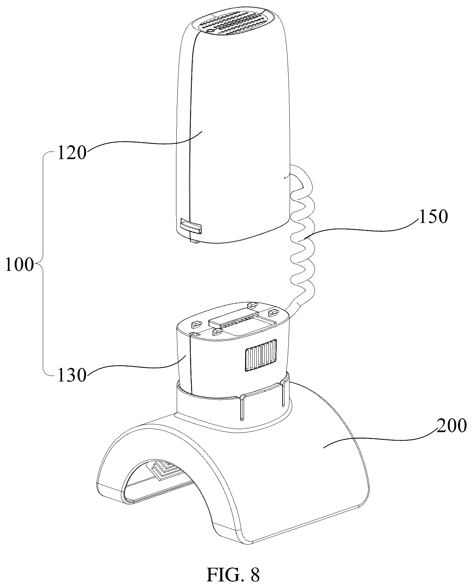

Referring to FIG. 8, in other embodiments, the main body 100 is of a split structure, and the power supply component and the light-emitting component 110 are provided separately. Specifically, the main body 100 may include a first housing 120 and a second housing 130, and the power supply component and the light-emitting component 110 are respectively provided in the first housing 120 and the second housing 130.

The power supply component is accommodated in the first housing 120, and they constitute the body part of the main body 100. The light-emitting component 110 is accommodated in the second housing 130, and they constitute the lamp head part of the main body 100.

The first housing 120 and the second housing 130 may be provided separately; or, the first housing 120 and the second housing 130 may also be detachably connected.

As shown in FIG. 8, the first housing 120 and the second housing 130 are separately provided, and they are connected by a connection cable 150. That is to say, in this embodiment, the body part and the lamp head part are not directly connected, but they are physically connected and electrically connected through the connection cable 150. Specifically, one end of the connection cable 150 is physically and electrically connected to the body part, and the other end is physically and electrically connected to the lamp head part.

Optionally, the connection cable 150 and the body part may be detachably connected. Or the connection cable 150 and the body part may be fixedly connected.

Optionally, the connection cable 150 and the lamp head part may be detachably connected. Or the connection cable 150 and the lamp head part may be fixedly connected.

Optionally, the body part where the power supply component is located may be connected to head parts where two or more light-emitting components 110 are located. Specifically, the body part may be connected to a plurality of connection cables 150, and each connection cable 150 is respectively connected to a lamp head part.

In this embodiment, by arranging the power supply component and the light-emitting component 110 of the main body 100 separately, when using the beauty device, only the lamp head part where the light-emitting component 110 is located needs to be connected to the wearable accessory 200. On the one hand, the weight of the lamp head part is small, which can reduce the load and improve the use comfort. On the other hand, one body part can be connected to more than one lamp head part, which can achieve more convenient use and operation, make the whole machine more flexible, and further improve the user experience.

**In** other embodiments, the first housing 120 and the second housing 130 are detachably connected. That is to say, in this embodiment, the body part and the lamp head part can be detachably connected, and after the body part and the lamp head part are mechanically connected, they are electrically connected at the same time. When using the beauty device, the lamp head part where the light-emitting component 110 is located needs to be connected with the body part as a whole, and then the whole is connected with the wearable accessory 200.

The first housing 120 and the second housing 130 may be mechanically connected by using various structures that can realize detachable fixed connection, such as a threaded locking structure, a snapping and buckling structure, an interference fit structure, or a magnetic attraction structure, etc. In addition, the body part maybe electrically connected with the lamp head part by using a plug-in terminal structure or an electrical contact structure.

In the embodiment of the present application, the power supply component may include a control circuit board and a battery, the battery is connected with the control circuit board, and the battery may supply power to the entire beauty device 10. Optionally, the battery is a rechargeable battery, such as a lithium battery.

In other embodiments, the power supply component may not include a battery, and the beauty device 10 is used by being connected to an external commercial power or an external storage battery when working.

In the embodiment of the present application, the light-emitting component 110 includes a pulsed light source capable of emitting pulsed light, and the pulsed light source may be a xenon lamp.

It can be understood that for those skilled in the art, equivalent substitutions or changes can be made according to the technical solution of the present application and its inventive concept, and all these changes or substitutions should belong to the protection scope of the claims attached to the present application.

## Claims

1. A beauty device, **characterized by** comprising:
a main body provided with a power supply component for power supply and a light-emitting component comprising a pulsed light source for emitting pulsed light; and
a wearable accessory comprising a housing adapted to be worn on a corresponding part of a human body;
wherein a cavity for the corresponding part of the human body to enter is formed inside the housing, and at least one light inlet is provided on the housing; and
the main body is detachably connected to the at least one light inlet of the housing, and pulsed light emitted by the pulsed light source enters the cavity inside the housing through the at least one light inlet to act on a human skin.

2. The beauty device according to claim 1, further comprising an electrical connection assembly provided on a surface where the main body and the housing are docked;
the electrical connection assembly comprises a first electrical connection portion and a second electrical connection portion that are electrically connected in a matching manner; and
the first electrical connection portion is provided on one of the main body and the housing, and the second electrical connection portion is provided on another of the main body and the housing.

3. The beauty device according to claim 2, further comprising:
a heating module provided inside the housing for raising a temperature of the human skin;
a temperature sensor element provided on a surface of the housing corresponding to the human skin for sensing skin temperature; and
an image acquisition module provided inside the housing for acquiring skin state information;
wherein the heating module, the temperature sensor element, and the image acquisition module are electrically connected to the power supply component on the main body through the electrical connection assembly.

4. The beauty device according to claim 1, further comprising a snap assembly for snapping and fixing the main body and the housing;
wherein the snap assembly comprises a snap-fit portion and a snap-in portion that are in snapping fit;
the snap-fit portion is provided on one of the main body and the housing, and the snap-in portion is provided on another of the main body and the housing.

5. The beauty device according to claim 4, wherein the snap-fit portion is a snap seat protruding from a surface of the housing, the snap seat is provided with a plug-in slot perpendicular to each light inlet, and a snap protrusion is protruded from an inner wall of the plug-in slot; and
the snap-fit portion is a snap groove opened on a circumferential side wall of the main body, and when the main body is inserted into the snap seat along the plug-in slot, the snap protrusion is snapped in the snap groove.

6. The beauty device according to claim 4, wherein the snap-fit portion is a snap seat protruding from a surface of the housing, one side of the snap seat is open to form a sliding groove parallel to each light inlet, and a snap protrusion is protruded from an inner wall of the sliding groove; and
the snap-in portion is a snap groove opened on a circumferential side wall of the main body, and when the main body slides into the snap seat along the sliding groove, and the snap protrusion is snapped in the snap groove.

7. The beauty device according to claim 6, wherein a limitation portion is provided on the housing, and located at an opening of the snap seat, for limiting the main body in the sliding groove.

8. The beauty device according to claim 1, wherein a reflective layer is provided on a surface of the housing corresponding to the human skin, and distributed around each light inlet.

9. The beauty device according to claim 1, wherein a filter is detachably provided at each light inlet of the housing, and the pulsed light emitted by the pulsed light source is transmitted through the filter.

10. The beauty device according to claim 1, further comprising an LED light source provided on a surface of the housing corresponding to the human skin.

11. The beauty device according to claim 1, further comprising an LED light source provided on the main body and capable of extending into the housing through each light inlet.

12. The beauty device according to claim 1, wherein the housing comprises a housing body and an abutting portion provided on a circumferential edge of the housing body, the abutting portion is integrally connected with the housing body, and the abutting portion is configured to make the cavity inside the housing relatively closed when the wearable accessory is worn on the corresponding part of the human body.

13. The beauty device according to claim 1, wherein a binding member is provided on the housing, and detachably connected to the housing, for fixing the wearable accessory to the corresponding part of the human body.

14. The beauty device according to claim 1, wherein a binding member is provided on the housing, and fixed to the housing, for fixing the wearable accessory to the corresponding part of the human body.

15. The beauty device according to claim 1, wherein the wearable accessory is configured to cover at least one form of skin on a face, a neck, a limbs, an abdomen, and a back.

16. The beauty device according to claim 15, wherein each of a plurality of light inlets is provided corresponding to different skin areas of the human body.

17. The beauty device according to claim 1, wherein the main body is of an integrated structure.

18. The beauty device according to claim 1, wherein the main body is of a split structure, and the power supply component and the light-emitting component are provided separately.
